Europäisches Patentamt

(19) European Patent Office

Office européen des brevets

(11) Publication number: **0 362 294 B1**

# EUROPEAN PATENT SPECIFICATION

(45) Date of publication of patent specification: 30.06.93   (51) Int. Cl.⁵: **G01N 33/74**, G01N 33/76, G01N 33/68

(21) Application number: **88905972.1**

(22) Date of filing: **11.07.88**

(86) International application number:
**PCT/GB88/00557**

(87) International publication number:
**WO 89/00696 (26.01.89 89/03)**

(54) **ANTENATAL SCREENING FOR DOWN'S SYNDROME.**

(30) Priority: **09.07.87 GB 8716158**
**23.10.87 GB 8724913**

(43) Date of publication of application:
**11.04.90 Bulletin 90/15**

(45) Publication of the grant of the patent:
**30.06.93 Bulletin 93/26**

(84) Designated Contracting States:
**AT BE CH DE FR GB IT LI LU NL SE**

(56) References cited:
**EP-A- 158 973**
**WO-A-86/04418**
**US-A- 4 454 232**

(73) Proprietor: **3i RESEARCH EXPLOITATION LIMITED**
**91 Waterloo Road**
**London SE1 8XP(GB)**

(72) Inventor: **WALD, Nicholas, John**
**9 Park Crescent**
**London W1N 5HB(GB)**
Inventor: **CUCKLE, Howard, Stephen**
**35 Cromwell Avenue Highgate**
**London N6 5HN(GB)**
Inventor: **CANICK, Jacob, Abraham**
**32 Duncklee Street**
**Newton, MA 02161(US)**
Inventor: **HADDOW, James, Edward**
**P.O. Box 185**
**Sebago Lake, ME 04075(US)**

(74) Representative: **BATCHELLOR, KIRK & CO.**
**2 Pear Tree Court Farringdon Road**
**London EC1R 0DS (GB)**

**Description**

This invention relates to antenatal screening for risk of Down's Syndrome in a foetus.

Risk of Down's syndrome in a foetus is known to increase with the age of the mother and it is this fact which forms the basis for selection of mothers for further investigation. Further testing involves sampling of the amniotic fluid by amniocentesis, a procedure which is not completely free from risk to the mother or foetus, induction of miscarriage being a recognised hazard.

The amniotic fluid test for Down's syndrome (the recognition of an extra chromosome 21 in foetal cells) is diagnostic.

There is a need for a screening method which will identify pregnancies most at risk so as to justify amniocentesis with its attendant risks.

An object of the present invention is to provide an improved screening procedure.

According to the present invention there is provided a method of determining increased risk of Down's syndrome in a foetus, comprising assaying a sample of maternal serum, taken before the beginning of the third trimester of pregnancy, for at least one serum constituent selected from;

unconjugated oestriol;

progesterone;

16-alpha-hydroxy-dehydroepiandrosterone sulphate (16-alpha-hydroxy-DHEAS);

dehydroepiandrosterone sulphate (DHEAS), and precursors or metabolites thereof, comparing the amount of the constituent(s) assayed with a control value derived from pregnancies unaffected by Down's Syndrome,

the results of said comparison being indicative of increased risk of foetal Down's Syndrome.

The method may also include an additional assay for alpha-fetoprotein.

It is preferred that the method include a further assay for human chorionic gonadotrophin (hGC).

In a particularly preferred method three assays are carried out for alpha-fetoprotein (AFP), unconjugated oestriol and human chorionic gonadotrophin.

In another preferred embodiment the method includes additional assays for either or both of

16-alpha-hydroxy-dehydroepiandrosterone sulphate (16-alpha-hydroxy-DHEAS); and,

dehydroepiandrosterone sulphate (DHEAS).

In deriving the risk indicator the assay results may be interpreted in combination with maternal age.

The present invention also provides an assay kit comprising means for conducting the assays for metabolites identified above and means for producing from the said assay results an indication of increased risk of foetal Down's syndrome.

This invention is based on the fact that serum AFP, and unconjugated oestriol in maternal blood in early pregnancy are significantly lower in affected pregnancies than unaffected pregnancies, and therefore their precursors including 16-alpha- hydroxy DHEAS and DHEAS are also affected, and serum progesterone and human chorionic gonadotrophin are both greater in affected pregnancies than in unaffected pregnancies.

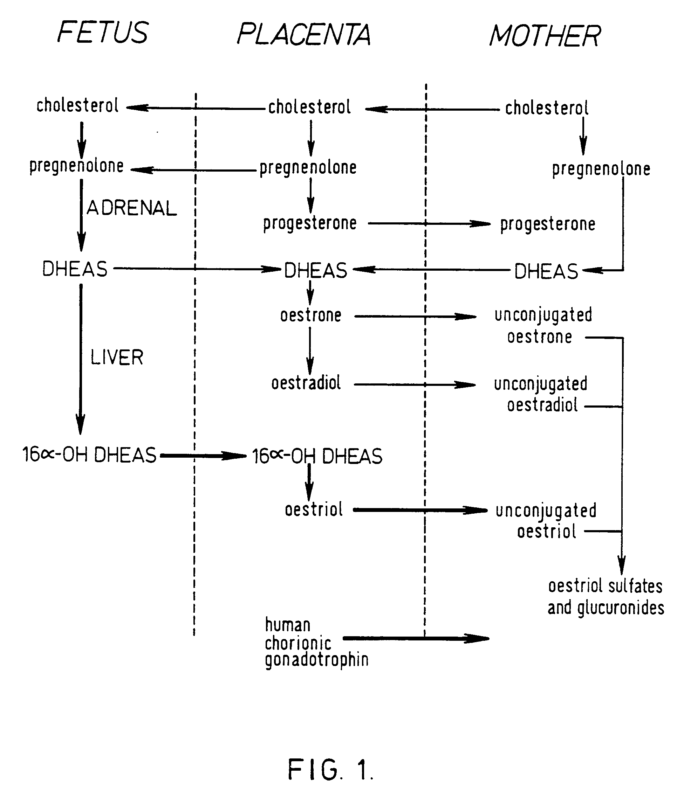

The abnormal unconjugated oestriol, progesterone and AFP levels in Down's syndrome pregnancies reflect abnormal synthesis metabolism in the foetus and/or placenta. Fig.1 of the accompanying drawing sets forth the metabolic pathways relating to the biosynthesis of oestriol (and progesterone) during pregnancy, the oestriol biosynthesis route being shown in bold type. The abbreviations DHEAS means dehydroepiandrosterone sulphate and 16-OH DHEAS means its 16-hydroxy-derivative.

AFP is manufactured by the yolk sac and the foetal liver.

Thus, as shown in Fig.1, oestriol, derived from cholesterol of maternal origin, is converted, via pregnenolone and DHEAS to 16-hydroxy-DHEAS which is then converted by placental activity to oestriol which returns, in unconjugated form, into maternal serum where, subsequently, it is converted to oestriol sulphates and glucuronides.

Progesterone arises by placental activity which converts cholesterol to pregnenolone and hence to progesterone, a portion of the pregnenolone being passed to the foetus, for conversion to oestriol by the route described above, and the remainder returned to maternal serum.

The median maternal serum unconjugated oestriol level at 13 to 27 weeks gestation in seventy-seven pregnancies associated with Down's syndrome was 0.73 multiples of the median (MoM) value for 385 unaffected control pregnancies matched for maternal age, gestational age, and duration of storage of the serum sample. This result was statistically highly significant. Serum unconjugated oestriol is a better discriminator between Down's syndrome and unaffected pregnancies than maternal age or serum AFP. The extent of the discrimination was also independent of maternal age and largely independent of serum AFP. When used in combination, the three variables, age, serum AFP and serum unconjugated oestriol, were more effective than any one by itself or two in combination at identifying Down's syndrome. The inclusion of serum progesterone leads to significant increase of the effectiveness of the detection.

The median maternal serum progesterone level at 13 to 27 weeks gestation in 77 pregnancies associated with Down's syndrome was 1.19 multiples of the median value (MoM) for 385 unaffected control pregnancies of the same gestational age. The increase in values was statistically significant ($X^2_1$ = 14, based on analysis of variance of the ranks of matched sets). There was more overlap in the distribution of progesterone between affected and unaffected pregnancies than was the case for unconjugated oestriol.

In normal pregnancy the foetal adrenal cortex produces DHEAS which enters the foetal circulation and passes to the foetal liver where most of it is undergoes 16-alpha-hydroxylation. The newly formed 16-alpha-hydroxy-DHEAS reaches the placenta where a portion of it converted in four enzymatic steps to oestriol. Unconverted 16-alpha-hydroxy-DHEAS is also likely to pass into the mother's circulation. It is possible that in maternal serum almost all of the 16-alpha-hydroxy-DHEAS is derived from the foetus rather than from the mother, and therefore measurement of this substance is a specific marker of foetal development.

Down's syndrome foetuses are less mature than unaffected foetuses and so pregnancies associated with foetal Down's syndrome would be expected to have lower 16-alpha-hydroxy-DHEAS levels than unaffected pregnancies. It is by similar reasoning that lower levels of DHEAS will be found in the maternal blood of women carrying foetuses with Down's syndrome than in those carrying unaffected foetuses.

Therefore, either or both of DHEAS and 16-alpha-hydroxy-DHEAS provide useful markers of Down's syndrome in early pregnancy, particularly when the results are viewed in combination with the other indicators referred to herein.

The method of the present invention increases the efficiency of screening of women for Down's syndrome by identifying a greater proportion of women with affected pregnancies as eligible for amniocentesis ( i.e. increasing the proportion of true positives) without also increasing the proportion of women with unaffected pregnancies who, by some criteria, would be eligible for amniocentesis (i.e. without increasing the proportion of false positives).

The invention will now be described, by way of illustration, in the following description.

## Methods

Using a -40ºC antenatal serum bank relating to over 30,000 pregnancies, 77 antenatal serum samples were identified from 77 singleton pregnancies associated with Down's syndrome (cases). Each sample was matched with a stored sample from five unaffected pregnancies (controls) matching for maternal age (within the same year) gestational age (within one week) and duration of storage (within one year) of the sample. The samples were retrieved from storage for unconjugated oestriol and AFP analysis. None of the cases or controls were from pregnancies associated with a neural tube defect, and all the samples were collected between 13 and 27 completed weeks of gestation.

Unconjugated oestriol was assayed using a direct, non-extraction radio-immunoassay (Amerlex oestriol RIA kit, Amersham). The assay was modified by doubling the sample volume to 40 microlitres for all the specimens analysed.

Maternal serum AFP was assayed an immunoradiometric assay (Boots-Celltech Diagnostics Limited).

Maternal serum progesterone was assayed using a radiometric assay (Amerlex -M progesterone (New) kit, Amersham) and serum DHEAS also by radiometric assay (Pantex DHEA-S $I^{125}$ kit).

All the assays were performed within about one week. Cases and controls were always assayed in the same analytical batch, and the assays were done without knowledge of whether they were from affected or unaffected pregnancies.

Maternal serum hCG was assayed using an immunoradiometric assay (Serono-MAIA-clone kit calibrated to the first international reference preparation) at a 1 in 500 dilution. All the assays were performed within three days and cases an controls were always assayed in the same analytical batch without knowledge of whether the samples were from affected ot unaffected pregnancies.

## Results

## Maternal Serum Unconjugated Oestriol

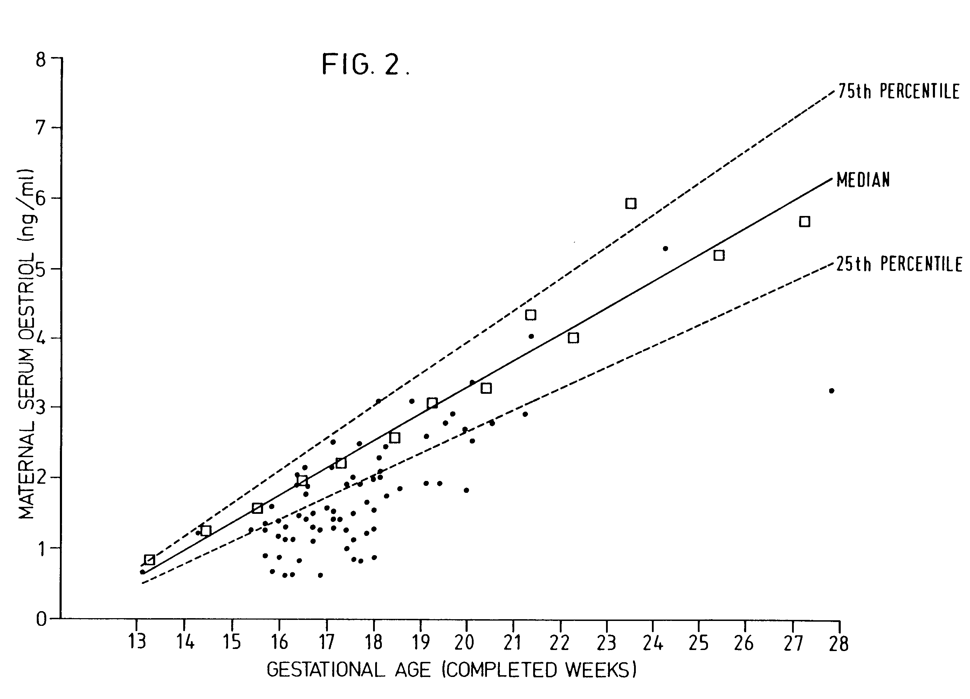

Fig.2 shows the individual unconjugated oestriol levels for each pregnancy associated with Down's syndrome, together with the median and the 25th and 75th percentile values for unaffected pregnancies, based on linear regressions on gestation weighted for the number of women tested in each week of gestation. The observed medians for unaffected pregnancies at each week of gestation are also shown. On average, the unconjugated oestriol values were significantly lower for the Down's syndrome pregnancies than for the unaffected pregnancies (p < 0.001, by analysis of variance of the ranks within matched sets). There was no tendency for the difference to be materially different in any particular week from 13 to 27 weeks of gestation.

Unconjugated oestriol levels can conveniently be expressed in multiples of the median for unaffected pregnancies of the same gestational age (MoM) using the regressed medians shown in Table I below.

TABLE I

| Percentile | MSO (MoM) | |
|---|---|---|
| | Down's syndrome pregnancies (n = 77) | Unaffected pregnancies (n = 385) |
| 25th | 0.59 | 0.83 |
| 50th (median) | 0.73 | 1.00 |
| 75th | 0.90 | 1.19 |
| MSO = maternal serum unconjugated oestriol | | |

Table I shows the 25th, 50th and 75th percentiles of maternal serum unconjugated oestriol in MoMs for the Down's syndrome and unaffected pregnancies. The median serum AFP for the affected pregnancies was only 73% of the value for unaffected pregnancies (p < 0.001).

Table II shows the proportion of Down's syndrome and unaffected pregnancies with median serum unconjugated oestriol levels less than or equal to specified cut-off levels. Gaussian frequency distributions fitted the data reasonably well.

TABLE II

| Unconjugated Oestriol Level (MoM) | Down's syndrome pregnancies (%) | Unaffected pregnancies (%) |
|---|---|---|
| ≤ 0.40 | 9.1 | 1.3 |
| ≤ 0.50 | 16 | 3.1 |
| ≤ 0.60 | 26 | 4.9 |
| ≤ 0.70 | 46 | 9.6 |
| ≤ 0.80 | 62 | 21 |
| ≤ 0.90 | 75 | 35 |
| ≤ 1.00 | 86 | 50 |

Table III shows the median maternal serum unconjugated oestriol (MSO) level in Down's syndrome and unaffected pregnancies according to maternal age . In both Down's syndrome and unaffected pregnancies the median serum unconjugated oestriol provides a measure of the risk of Down's syndrome that is independent of maternal age.

TABLE III

| Maternal age at expected date of delivery. (years) | Down's syndrome pregnancies MSO | | Unaffected pregnancies MSO | |
|---|---|---|---|---|
| | (MoM) | Number | (MoM) | Number |
| < 30 | 0.75 | 20 | 0.98 | 100 |
| 30-34 | 0.72 | 17 | 1.04 | 85 |
| 35-39 | 0.76 | 19 | 1.00 | 95 |
| 40 or more | 0.73 | 21 | 1.00 | 105 |
| All | 0.73 | 77 | 1.00 | 385 |

4

Table IV shows that in both Down's syndrome and unaffected pregnancies the median maternal serum unconjugated oestriol level (MSO) increases with increasing maternal serum AFP level expressed like unconjugated oestriol in MoMs). The correlation between unconjugated oestriol and the log of AFP levels is statistically significant ($r = 0.21$, $p < 0.05$ for Down's syndrome pregnancies and $r = 0.20$, $p < 0.001$ for unaffected pregnancies). A bivariate Gaussian frequency distribution fitted the data reasonably well.

TABLE IV

| Serum AFP level (MoM) | Down's syndrome pregnancies MSO | | Unaffected pregnancies MSO | |
|---|---|---|---|---|
| | (MoM) | Number | (MoM) | Number |
| ≤ 0.60 | 0.66 | 17 | 0.74 | 22 |
| 0.61-0.80 | 0.72 | 24 | 0.95 | 76 |
| 0.81-1.00 | 0.82 | 23 | 0.96 | 94 |
| > 1.00 | 0.82 | 13 | 1.04 | 193 |
| All | 0.73 | 77 | 1.00 | 385 |

A way of combining information on median serum AFP and median serum unconjugated oestriol levels which gives equal weight to both tests is to regard a screening result as positive if either level is less than or equal to the same percentile of normal. Using this approach, for a given number of affected pregnancies with positive results there were fewer unaffected pregnancies with positive results when both tests were used than when each test was considered alone (Table V).

TABLE V

| Downs Syndrome Detectn. (%) | False-positive rate (%) using | | | | | |
|---|---|---|---|---|---|---|
| | Age alone (a) | AFP alone (b) | $uE_3$ alone (c) | Age & AFP (d) | Age & $uE_3$ (e) | Age, AFP and $uE_3$ (f) |
| 60 | 31 | 33 | 28 | 20 | 14 | 12 |
| 55 | 25 | 28 | 19 | 16 | 11 | 9.1 |
| 50 | 19 | 24 | 16 | 12 | 8.8 | 7.0 |
| 45 | 15 | 20 | 13 | 9.8 | 6.7 | 5.3 |
| 40 | 11 | 17 | 11 | 7.3 | 5.0 | 3.9 |
| 35 | 7.5 | 14 | 8.5 | 5.3 | 3.7 | 2.8 |
| 30 | 5.1 | 11 | 6.6 | 3.6 | 2.5 | 1.9 |
| 25 | 3.3 | 8.2 | 5.0 | 2.2 | 1.6 | 1.2 |
| 20 | 1.9 | 6.0 | 3.5 | 1.3 | 0.9 | 0.7 |

Note:
A result is positive if (a) age is advanced, (b) AFP is low, (c) $uE_3$ is low or, for (d), (e) and (f), the risk of Downs Syndrome (at term) is high.

Most of the saving achieved by doing both tests (AFP and MSO) compared to only one was due to the screening efficiency of unconjugated oestriol rather than AFP. For example, to identify 35% of Down's syndrome pregnancies as positive (see Table V), the use of both tests reduced the proportion of unaffected pregnancies with positive results by only 0.1% compared to that achieved with unconjugated oestriol alone but reduced the proportion by 2.2% compared to that achieved with AFP alone. Logistic regression analysis confirmed that unconjugated oestriol was a stronger predictor for Down's syndrome than AFP, though each provided additional information that was statistically significant ($X_1^2 = 45.4$, $p < 0.0001$ for unconjugated oestriol added to AFP; $X_1^2 = 11.2$, $p < 0.001$ for AFP added to unconjugated oestriol).

These results confirm that maternal serum unconjugated oestriol is, on average, lower in pregnancies associated with Down's syndrome than unaffected pregnancies, an effect which is independent of maternal age. The results also show that a serum unconjugated oestriol level distinguished affected from unaffected pregnancies more effectively than a serum AFP level.

5

The explanation for the low maternal serum unconjugated oestriol levels in Down's syndrome pregnancies is unknown. Fig.1 shows the metabolic pathway of oestriol biosynthesis in the foetus and placenta and indicates the sites at which a metabolic alteration could arise. In normal pregnancy, the foetal adrenal cortex produces DHEAS which enters the foetal circulation and passes to the foetal liver, where most of it undergoes 16-alpha- hydroxylation. The newly formed 16-hydroxy-DHEAS reaches the placenta where it is converted in four enzymatic steps (sulphatase, aromatase and 17-beta-hydroxysteroid dehydrogenase) to oestriol. Oestriol then diffuses into the maternal compartment, where it can be measured as an unconjugated steroid, its rise in concentration paralleling the growth of the foetus and the placenta. Unlike total oestriol, unconjugated oestriol in maternal serum is entirely derived from the foetus and the placenta. For this reason it use herein as a more sensitive indication of altered foetal metabolism that the total maternal serum oestriol.

The introduction into the screening programme of a progesterone assay, giving four parameters as indicators of risk (maternal age, unconjugated oestriol, AFP and progesterone) further increases the degree of selectivity obtained.

It has also been found to be advantageous to include in the screening programme an assay for human chorionic gonadotrophin (hCG). The median maternal hCG level in seventy-seven pregnancies associated with Down Syndrome was twice the median level in 385 unaffected pregnancies matched for maternal age, gestational age and duration of serum sample storage ($p < 0.001$). Maternal serum hCG measurement was therefore an effective screening test for Down's Syndrome. For example, to achieve a 30% detection rate, the false-positive (the proportion of unaffected pregnancies classified as positive) was 3% for hCG, lower than for maternal age (5%) and unconjugated oestriol (7%) and AFP (11%). The most effective screen was achieved by taking account of all four of these parameters; to achieve the same 30% detection rate, the false-positive rate declined to 0.5%.

Combining Screening Tests

When using several variables in combination to screen for a particular disorder it is necessary to assess the extent of the correlation between the variables concerned. If two variables are perfectly correlated one adds nothing to the other in assessing a subject's risk of having the disorder; if they are completely unrelated each provides an independent measure of risk. To the extent that they may be partially correlated there will be some independent information. The associations between hCG, $uE_3$ and AFP in affected and unaffected pregnancies are shown in Table VI below.

EP 0 362 294 B1

## TABLE VI

| | Down's Syndrome Pregnancies Median (No) hCG (MoM) | Unaffected Pregnancies Median (No) hCG (MoM) |
|---|---|---|
| Age (years) | | |
| < 30 | 1.94 (20) | 1.10 (100) |
| 30- | 1.62 (17) | 0.96 (85) |
| 35- | 2.11 (19) | 0.95 (95) |
| ≥ 40 | 2.19 (21) | 1.02 (105) |
| uE$_3$ (MoM) | | |
| < 0.60 | 2.71 (20) | 1.25 (19) |
| 0.60- | 1.88 (20) | 1.21 (36) |
| 0.75- | 1.85 (18) | 0.97 (77) |
| ≥ 0.90 | 1.84 (19) | 1.00 (253) |
| AFP (MoM) | | |
| < 0.60 | 1.64 (17) | 1.02 (22) |
| 0.60- | 2.05 (24) | 0.91 (75) |
| 0.80- | 2.12 (23) | 0.89 (95) |
| ≥ 1.00 | 2.24 (13) | 1.10 (193) |
| All | 2.04 (77) | 1.02 (385) |

These results indicate no evidence of an association between hCG levels and maternal age, but there is a small negative association between hCG and unconjugated oestriol (uE$_3$) levels. The relationship between hCG and AFP is less evident, but since uE$_3$ and AFP have previously been shown to be associated, and hCG and uE$_3$ are, there is probably an underlying association.

Table VII shows the risk of having a Down's Syndrome pregnancy according to selected values of the three biochemical screening tests (maternal serum AFP, unconjugated oestriol and hCG) as they apply to a 35 year old woman.

TABLE VII

| uE$_3$ (MoM) | hCG (MoM) | AFP (MoM) | | |
|---|---|---|---|---|
| | | 0.4 | 1.0 | 2.5 |
| 0.4 | 0.5 | 1:370 | 1:2800 | 1:22000 |
| | 1.0 | 1: 84 | 1: 480 | 1: 2800 |
| | 2.0 | 1: 16 | 1: 69 | 1: 310 |
| 1.0 | 0.5 | 1:820 | 1:4800 | 1:28000 |
| | 1.0 | 1:330 | 1:1400 | 1: 6400 |
| | 2.0 | 1:110 | 1: 360 | 1: 1200 |
| 1.4 | 0.5 | 1:2200 | 1:11000 | 1:52000 |
| | 1.0 | 1:1300 | 1: 4600 | 1:17000 |
| | 2.0 | 1: 630 | 1: 1700 | 1: 4700 |

7

The results in Table VII show that if the results of any two tests are known, the third is still informative. For example, if a 35 year old woman had an AFP level of 1.0 MoM and an $uE_3$ level of 0.4 MoM, the risk would be 1:2800 at a hCG level of 0.5 MoM and 1:69 at 2.0 MoM hCG.

If only two tests are to be carried out, AFP and hCG are the preferred pair since AFP is of separate value in screening for neural tube defects and the performance of AFP and hCG in combination is not much worse than that using $uE_3$ and hCG in combination. The best overall results however are obtained using all three tests, together with maternal age. This, for example, yielded a detection rate of 60% with a false-positive rate of 4.7% (see below).

Table VIII summarises the rate of false positive detection corresponding to Down's Syndrome detection rates using maternal age in conjuction with assays for unconjugated oestriol ($uE_3$), AFP, and hGC alone or in combination.

TABLE VIII

| Down Syndrome Detection Rate (%) | False-positive rate (%) using age with: | | | | | | |
|---|---|---|---|---|---|---|---|
| | AFP | $uE_3$ | hCG | AFP and $uE_3$ | AFP and hCG | $uE_3$ and hCG | AFP, $uE_3$ and hCG |
| 80 | 44 | 34 | 27 | 29 | 20 | 20 | 16 |
| 75 | 37 | 27 | 21 | 23 | 15 | 15 | 12 |
| 70 | 30 | 22 | 16 | 18 | 12 | 11 | 8.6 |
| 65 | 25 | 18 | 12 | 15 | 8.8 | 8.1 | 6.4 |
| 60 | 20 | 14 | 9.5 | 12 | 6.7 | 6.0 | 4.7 |
| 55 | 16 | 11 | 7.2 | 9.1 | 5.0 | 4.4 | 3.4 |
| 50 | 12 | 8.8 | 5.4 | 7.0 | 3.7 | 3.2 | 2.5 |
| 45 | 9.7 | 6.7 | 3.9 | 5.3 | 2.7 | 2.3 | 1.7 |
| 40 | 7.3 | 5.0 | 2.8 | 3.9 | 1.9 | 1.6 | 1.2 |
| 35 | 5.3 | 3.7 | 1.9 | 2.8 | 1.3 | 1.0 | 0.8 |
| 30 | 3.6 | 2.5 | 1.2 | 1.9 | 0.8 | 0.7 | 0.5 |
| 25 | 2.2 | 1.6 | 0.8 | 1.2 | 0.5 | 0.4 | 0.3 |
| 20 | 1.3 | 0.9 | 0.4 | 0.7 | 0.3 | 0.2 | 0.2 |

Table IX below summarises the rate of detection at 5% false-positive rate using a variety of assays alone or in combination. The abbreviations used in the first column are as follows:

A = ($\log_{10}$) alpha-fetoprotein (AFP); E = unconjugated oestriol; H = ($\log_{10}$) human chorionic gonadotrophin (hCG); P = ($\log_{10}$) progesterone

TABLE IX

| Meas | d | Without DHEAS | | | d | With DHEAS | | |
|---|---|---|---|---|---|---|---|---|
| | | Risk | %Det | PPV | | Risk | %Det | PPV |
| AEHP | 1.771 | 232 | 63.6 | 62 | 1.795 | 232 | 64.3 | 61 |
| AEH | 1.680 | 227 | 60.6 | 65 | 1.700 | 228 | 61.3 | 64 |
| EHP | 1.601 | 226 | 58.1 | 68 | 1.632 | 226 | 59.1 | 67 |
| AEP | 1.585 | 225 | 57.5 | 68 | 1.619 | 226 | 58.7 | 67 |
| EH | 1.539 | 225 | 56.1 | 70 | 1.565 | 225 | 56.9 | 69 |
| AHP | 1.517 | 225 | 55.4 | 71 | 1.546 | 225 | 56.3 | 70 |
| AH | 1.493 | 225 | 54.6 | 72 | 1.519 | 225 | 55.5 | 71 |
| EP | 1.390 | 227 | 51.4 | 76 | 1.434 | 226 | 52.8 | 75 |
| HP | 1.272 | 231 | 47.9 | 82 | 1.312 | 229 | 49.1 | 80 |
| H | 1.268 | 231 | 47.8 | 82 | 1.305 | 229 | 48.8 | 80 |
| AE | 1.159 | 237 | 44.7 | 88 | 1.193 | 235 | 45.6 | 86 |
| AP | 1.080 | 243 | 42.6 | 92 | 1.133 | 239 | 44.0 | 89 |
| E | 1.006 | 249 | 40.7 | 96 | 1.051 | 246 | 41.9 | 94 |
| A | 0.703 | 285 | 34.7 | 113 | 0.767 | 277 | 35.8 | 110 |
| P | 0.656 | 291 | 34.0 | 116 | 0.753 | 278 | 35.5 | 111 |
| DHEAS | - | - | - | - | 0.333 | 321 | 30.7 | 128 |

d = distance, in standard deviation units, between affected and unaffected groups.

Risk is quoted as a ratio of 1: the figure in the Table.

PPV = positive predictive value and is an indicator of the odds of the actual occurrence of Down's Syndrome in the foetus. It is quoted as a ratio of 1: the figure in the Table.

In many existing screening programmes about 5% of women are selected for amniocentesis on the basis of advanced age alone. The method of this invention using assays for AFP, hCG, and unconjugated oestriol, in combination with maternal age detects over 60% of affected pregnancies, more than double that achievable with the same amniocentesis rate in existing screening programmes. As an illustration, this method has the potential to reduce the number of Down Syndrome births in the United Kingdom from about 900 per year, in the absence of screening, to about 350 per year.

The rate of detection may be further enhanced by ultrasound screening, using, for example, fetal femur length measurement as a complementary technique.

**Claims**

1. A method of determining increased risk of Down's syndrome in a foetus, comprising assaying a sample of maternal serum, taken before the beginning of the third trimester of pregnancy, for at least one serum constituent selected from;
   unconjugated oestriol;
   progesterone;
   16-alpha-hydroxy-dehydroepiandrosterone sulphate (16-alpha-hydroxy-DHEAS);
   dehydroepiandrosterone sulphate (DHEAS), and precursors or metabolites thereof, comparing the amount of the constituent(s) assayed with a control value derived from pregnancies unaffected by Down's Syndrome,
   the results of said comparison(s) being indicative of increased risk of foetal Down's Syndrome.

2. A method as claimed in claim 1 in which assays and control comparisons are conducted for more than one of said serum constituents.

3. A method as claimed in claim 2 in which assays and control comparisons are conducted, in combination, for at least unconjugated oestriol and progesterone.

4. A method as claimed in claim 2 in which assays and control comparisons are conducted, in combination, for at least unconjugated oestriol and DHEAS and/or 16-alpha-hydroxy-DHEAS.

5. A method as claimed in claim 2 in which assays and control comparisons are conducted, in combination, for at least unconjugated oestriol, progesterone and DHEAS.

6. A method as claimed in any preceding claim which includes an additional assay, and control comparison for alpha-fetoprotein.

7. A method as claimed in any preceding claim, which includes an additional assay and control comparison for human chorionic gonadotrophin.

8. A method as claimed in claim 7, in which assays and control comparisons are conducted for alpha-fetoprotein (AFP), unconjugated oestriol and human chorionic gonadotrophin.

9. A method as claimed in claim 8, including additional assay and control comparison for either or both of
16-alpha-hydroxy-dehydroepiandrosterone sulphate (16-alpha-hydroxy-DHEAS); and,
dehydroepiandrosterone sulphate (DHEAS).

10. A method as claimed in any preceding claim in which said comparison and a maternal age factor are indicative of said risk.

11. A method of determining increased risk of Down's Syndrome in a foetus, comprising assaying a sample of maternal serum, taken before the beginning of the third trimester of pregnancy, for alpha-fetoprotein, unconjugated oestriol and at least one serum constituent selected from;
progesterone;
human chorionic gonadotrophin;
16-alpha-hydroxy-dehydroepiandrosterone sulphate (16-alpha-hydroxy-DHEAS); and
dehydroepiandrosterone sulphate (DHEAS); and
precursors or metabolites thereof,
comparing the amount of the constituents assayed with control values thereof derived from pregnancies unaffected by Down's Syndrome,
the results of said comparison(s) being indicative of increased risk of foetal Down's Syndrome.

12. Assay means, useful in carrying out a method as claimed in claim 2, of determining increased risk of Down's Syndrome in a foetus, comprising in combination at least two means for assaying a sample of maternal serum, taken before the beginning of the third trimester of pregnancy, for more than one serum constituent selected from;
unconjugated oestriol;
progesterone;
16-alpha-hydroxy-dehydroepiandrosterone sulphate (16-alpha-hydroxy-DHEAS);
dehydroepiandrosterone sulphate (DHEAS), and precursors or metabolites thereof,
or which comprises means for assaying said sample for at least one serum constituent as defined herein in combination with means for assaying alpha-fetoprotein and/or human chorionic gonadotrophin.

13. Assay means as claimed in claim 12, comprising in combination means to assay at least unconjugated oestriol and progesterone.

14. Assay means as claimed in claim 12, comprising in combination means to assay at least unconjugated oestriol and DHEAS and/or 16-alpha-hydroxy-DHEAS.

15. Assay means as claimed in claim 12, comprising in combination means to assay unconjugated oestriol, progesterone and DHEAS.

16. Assay means as claimed in any one of claims 12 to 15, which further includes an assay for alpha-fetoprotein.

17. Assay means as claimed in any one of claims 12 to 16, which further includes an assay for human chorionic gonadotrophin.

**18.** Assay means as claimed in any one of claims 12 to 17, which includes means to assist derivation from the said assay results an indication of increased risk of foetal Down's Syndrome.

**19.** Assay means as claimed in claim 18 wherein said means to assist derivation comprise control values for the serum constituents which can be assayed by said means, which values have been derived from pregnancies unaffected by Down's Syndrome.

## Patentansprüche

**1.** Verfahren zur Bestimmung eines erhöhten Risikos für das Down-Syndrom in einem Fötus, bei dem man eine Probe Mutter-Serum, die vor dem Beginn des dritten Trimenons der Schwangerschaft genommen wurde, auf mindestens einen Serumbestandteil untersucht, der ausgewählt wird aus:

   unkonjugiertem Östriol;
   Progesteron;
   16-alpha-Hydroxydehydroepiandrosteronsulfat (16-alpha-Hydroxy-DHEAS);
   Dehydroepiandrosteronsulfat (DHEAS) und
   Vorläufern oder Metaboliten davon, wobei man die Menge des bzw. der untersuchten Bestandteile-(s) mit einem Kontrollwert vergleicht, der von Schwangerschaften stammt, die vom Down-Syndrom unbeeinflußt sind,
   wobei die Ergebnisse des bzw. der Vergleiche(s) ein erhöhtes Risiko für das Down-Syndrom im Fötus anzeigen.

**2.** Verfahren nach Anspruch 1, wobei man die Bestimmungen und die Kontrollvergleiche für mehr als einen der Serumbestandteile durchführt.

**3.** Verfahren nach Anspruch 2, wobei man die Bestimmungen und die Kontrollvergleiche in Kombination mindestens für unkonjugiertes Östriol und Progesteron durchführt.

**4.** Verfahren nach Anspruch 2, wobei man die Bestimmungen und die Kontrollvergleiche in Kombination mindestens für unkonjugiertes Östriol und DHEAS und/oder 16-alpha-Hydroxy-DHEAS durchführt.

**5.** Verfahren nach Anspruch 2, wobei man die Bestimmungen und die Kontrollvergleiche in Kombination mindestens für unkonjugiertes Östriol, Progesteron und DHEAS durchführt.

**6.** Verfahren nach einem der vorhergehenden Ansprüche, welches eine zusätzliche Bestimmung und einen Kontrollvergleich für alpha-Fetoprotein einschließt.

**7.** Verfahren nach einem der vorhergehenden Ansprüche, welches eine zusätzliche Bestimmung und einen Kontrollvergleich für Human-Choriongonadotrophin einschließt.

**8.** Verfahren nach Anspruch 7, wobei man die Bestimmungen und die Kontrollvergleiche für alpha-Fetoprotein (AFP), unkonjugiertes Östriol und Human-Choriongonadotrophin durchführt.

**9.** Verfahren nach Anspruch 8, welches eine zusätzliche Bestimmung und einen Kontrollvergleich für einen oder beide Vertreter aus

   16-alpha-Hydroxydehydroepiandrosteronsulfat (16-alpha-Hydroxy-DHEAS);und
   Dehydroepiandrosteronsulfat (DHEAS)
   einschließt.

**10.** Verfahren nach einem der vorhergehenden Ansprüche, wobei der Vergleich und ein mütterlicher Altersfaktor das Risiko anzeigen.

**11.** Verfahren zur Bestimmung eines erhöhten Risikos für das Down-Syndrom in einem Fötus, bei dem man eine Probe Mutter-Serum, die vor dem Beginn des dritten Trimenons der Schwangerschaft genommen wurde, auf alpha-Fetoprotein, unkonjugiertes Östriol und mindestens einen Serumbestandteil untersucht, der ausgewählt wird aus:

   Progesteron;
   Human-Choriongonadotrophin;

16-alpha-Hydroxydehydroepiandrosteronsulfat (16-alpha-Hydroxy-DHEAS;

Dehydroepiandrosteronsulfat (DHEAS); und

Vorläufern oder Metaboliten davon, wobei man die Menge der untersuchten Bestandteile mit einem Kontrollwert vergleicht, der von Schwangerschaften stammt, die vom Down-Syndrom unbeeinflußt sind, wobei die Ergebnisse des bzw. der Vergleiche(s) ein erhöhtes Risiko für das Down-Syndrom im Fötus anzeigen.

12. Untersuchungseinrichtung zum Durchführen eines Verfahrens gemäß Anspruch 2 zum Bestimmen eines erhöhten Risikos für das Down-Syndrom in einem Fötus, umfassend in Kombination mindestens zwei Einrichtungen zum Untersuchen einer Probe Mutter-Serum, die vor dem Beginn des dritten Trimenons der Schwangerschaft genommen wurde, auf mehr als einen Serumbestandteil, der ausgewählt ist aus;

unkonjugiertem Östriol;

Progesteron;

16-alpha-Hydroxydehydroepiandrosteronsulfat (16-alpha-Hydroxy-DHEAS;

Dehydroepiandrosteronsulfat (DHEAS), und

Vorläufern oder Metaboliten davon,

oder die eine Einrichtung umfaßt zum Untersuchen der Probe auf mindestens einen hier definierten Serumbestandteil in Kombination mit einer Einrichtung zum Untersuchen von alpha-Fetoprotein und/oder Human-Choriongonadotrophin.

13. Untersuchungseinrichtung nach Anspruch 12, aufweisend in Kombination eine Einrichtung zum Untersuchen zumindest von unkonjugiertem Östriol und Progesteron.

14. Untersuchungseinrichtung nach Anspruch 12, aufweisend in Kombination eine Einrichtung zum Untersuchen zumindest von unkonjugiertem Östriol und DHEAS und/oder 16-alpha-Hydroxy-DHEAS.

15. Untersuchungseinrichtung nach Anspruch 12, aufweisend in Kombination eine Einrichtung zum Untersuchen zumindest von unkonjugiertem Österiol, Progesteron und DHEAS.

16. Untersuchungseinrichtung nach einem der Ansprüche 12 bis 15, welche weiter eine Untersuchung von alpha-Fetoprotein einschließt.

17. Untersuchungseinrichtung nach einem der Ansprüche 12 bis 16, welche weiter eine Untersuchung von Human-Choriongonadotrophin einschließt.

18. Untersuchungseinrichtung nach einem der Ansprüche 12 bis 17, welche eine Einrichtung einschließt, die dabei hilft, aus den Untersuchungsergebnissen ein Anzeichen eines erhöhten Risikos für das Down-Syndrom im Fötus abzuleiten.

19. Untersuchungseinrichtung nach Anspruch 18, wobei die Hilfseinrichtung Kontrollwerte für die Serumbestandteile umfaßt, die mit dieser Hilfseinrichtung bestimmt werden können, wobei die Werte aus Schwangerschaften abgeleitet worden sind, die vom Down-Sysndrom unbeeinflußt sind.

**Revendications**

1. Procédé pour déterminer l'augmentation du risque d'un foetus de présenter le syndrome de Down (risque de trisomie 21) qui consiste à analyser un échantillon de sérum maternel, prélevé avant le troisième trimestre de grossesse, afin d'analyser l'un, au moins, des constituants suivants de celui-ci :

l'oestriol non-conjugué ;

la progestérone ;

le sulfate de 16-alpha-hydroxy-déhydroépiandrostérone (16-alpha-hydroxy-DHEAS) ;

le sulfate de déhydroépiandrostérone (DHEAS), et

ses précurseurs ou métabolites, qui consiste à comparer la quantité du ou des constituants analysés avec une grandeur témoin provenant de grossesses n'ayant pas été affectées par le syndrome de Down (risque de trisomie 21),

le résultat de cette ou de ces comparaisons fournissant une indication de l'augmentation du risque que le foetus présente le syndrome de Down (risque de trisomie 21).

**2.** Procédé selon la revendication 1, caractérisé en ce qu'on procède à ces analyses et à ces comparaisons de contrôle sur plus d'un constituant dudit sérum.

**3.** Procédé selon la revendication 2, caractérisé en ce qu'on procède auxdites comparaisons et auxdits contrôles, en combinaison, sur, au moins de l'oestriol non-conjugué et de la progestérone.

**4.** Procédé selon la revendication 2, caractérisé en ce qu'on procède aux analyses et aux comparaisons de contrôle, en combinaison, pour, au moins, un oestriol non-conjugué de DHEAS et/ou du 16-alpha-hydroxy-DHEAS.

**5.** Procédé selon la revendication 2, caractérisé en ce qu'on procède aux analyses et aux comparaisons, en combinaison, pour, au moins, l'oestriol non-conjugué, la progestérone et le DHEAS.

**6.** Procédé selon l'une quelconque des revendications précédentes, caractérisé par une analyse supplémentaire, et par une comparaison de contrôle de l'alpha-fétoprotéine.

**7.** Procédé selon l'une quelconque des revendications précédentes, caractérisé par une analyse et une comparaison de contrôle supplémentaires de la gonadotrophine chorionique humaine.

**8.** Procédé selon la revendication 7, caractérisé en ce qu'on procède aux analyses et aux comparaisons concernant les alpha-fétoprotéine (AFP), l'oestriol non-conjugué et la gonadotrophine humaine.

**9.** Procédé selon la revendication 8, caractérisé par une analyse et une comparaison de contrôle supplémentaires pour l'une et/ou l'autre des deux substances suivantes :
le sulfate de 16-alpha-hydroxy-déhydroépiandrostérone (16-alpha-hydroxy-DHEAS); et, le sulfate de déhydropiandrostérone (DHEAS).

**10.** Procédé selon l'une quelconque des revendications précédentes, caractérisé en ce que ladite comparaison et un facteur d'âge maternel sont les indicateurs dudit risque.

**11.** Procédé pour déterminer les risques que représente le syndrome de Down (risque de trisomie 21) dans un foetus, qui consiste à analyser un échantillon de sérum maternel, prélevé avant le troisième trimestre de grossesse, pour déterminer les alpha-fétoprotéines, l'oestriol non-conjugué et, au moins, l'un des constituants suivants du sérum choisi dans le groupe comprenant :
la progestérone ; la gonadotrophine chorionique humaine ; le sulfate de 16-alpha-hydroxy-déhydroépaindostérone (16-alpha-hydroxy-DHEAS) ; et leurs précurseurs ou métabolites, à comparer la quantité des constituants analysés avec leurs valeurs témoins provenant de grossesses non affectées par le syndrome de Down (risque de trisomie 21),
le résultat de ladite ou desdites comparaisons constituant une indication de l'augmentation du risque que le foetus présente le syndrome de Down (risque de trisomie 21).

**12.** Moyens d'analyse médicale, pour la mise en oeuvre du procédé spécifié sous 2, afin de déterminer l'accroissement du risque qu'un foetus présente le syndrome de Down (risque de trisomie 21), qui consiste à combiner, au moins, deux composants d'un échantillon de sérum maternel, prélevé avant commencement du troisième trimestre de la grossesse, choisis dans l'ensemble comprenant l'oestriol non-conjugué, la progestérone; le sulfate de 16-alpha-hydroxydéhydroépiandostérone (A6-alpha-hydroxy-DHEAS) et leurs précurseurs ou métabolites, ou qui comprend des moyens pour analyser ledit échantillon tel que spécifié ci-dessus en combinaison avec des moyens pour analyser l'alpha-fétoprotéine et/ou de la gonadotrophine chorionique humaine.

**13.** Moyens d'analyse tels que spécifiés dans la revendication 12, caractérisés en ce qu'ils comprennent, en combinaison, des moyens pour analyser, au moins, l'oestriol non-conjugué et la progestérone.

**14.** Moyens d'analyse selon la revendication 12, caractérisés en ce qu'ils sont combinés avec des moyens pour analyser, au moins, l'oestriol non-conjugué et le DHEAS et/ou le 16-alpha-hydroxy-DHEAS.

**15.** Moyens d'analyse tels que spécifiés dans la revendication 12, caractérisés en ce qu'ils comprennent, en combinaison, des moyens pour analyser l'oestriol non-conjugué, la progestérone et le DHEAS.

16. Moyens d'analyse tels que spécifiés dans l'une quelconque des revendications 12 à 15, caractérisés en ce qu'ils comprennent aussi des moyens pour rechercher les alpha-fétoprotéines.

17. Moyens d'analyse tels que spécifiés dans l'une quelconque des revendications 12 à 16, caractérisés par des moyens pour analyser la gonadotrophine chorionique humaine.

18. Moyens d'analyse selon l'une quelconque des revendications 12 à 17, caractérisés par des moyens pour extraire des variations ou des écarts desdits résultats d'analyse, des indications signalant une augmentation du risque que le foetus présente le syndrome de Down (risque de trisomie 21).

19. Moyens d'analyse selon la revendication 18, caractérisés en ce que lesdits moyens d'élaboration des variations ou des écarts comprennent des grandeurs nominales pour les constituants du sérum qui peuvent être analysées par lesdits moyens, lesquelles grandeurs ont été obtenues à partir de grossesses non-affectées par le syndrome de Down (risque de trisomie 21).

FIG. 1.

FIG. 2.

75th PERCENTILE

MEDIAN

25th PERCENTILE

MATERNAL SERUM OESTRIOL (ng/ml)

GESTATIONAL AGE (COMPLETED WEEKS)

EP 0 362 294 B1